# DEMANDE DE BREVET EUROPEEN

(11) **EP 3 009 085 A1**
(43) Date de publication de la demande: **20.04.2016**
(21) Numéro de dépôt: 14306628.0
(22) Date de dépôt: 14.10.2014
(51) Int. Cl.: A61B 17/221

(54) **Dispositif d'extraction d'un corps étranger d'un patient**

(71) Demandeur: Coloplast A/S, 3050 Humlebaek (DK)
(72) Inventeur: Torchio, Gerard Louis Florent, F-91370 Verrières le Buisson (FR); Scoffone, Cesare, 12051 Alba (Cuneo) (FR); Rigotto, David, 24200 Saint André d Allas (FR)

(57) **Abrégé**

La présente invention concerne un dispositif d'extraction (10) de corps étrangers d'un patient, ce dispositif étant caractérisé en ce qu'il comporte :
• un extracteur flexible (11) formé d'une gaine flexible (14), d'un mandrin de transmission (16) disposé dans ladite gaine flexible (14), d'une poignée (15) à laquelle est fixée une zone proximale dudit mandrin de transmission (16) et d'un outil lié à une extrémité distale dudit mandrin de transmission (16);
• un dispositif de rigidification (12) comportant un tube rigide (22) dans lequel peut être placée la gaine flexible (14) de l'extracteur flexible (11); et
• des moyens de connexion (13) de l'extracteur flexible (11) audit dispositif de rigidification (12).

La présente invention concerne également un dispositif de rigidification (12) destiné à coopérer avec un extracteur flexible (11), ce dispositif de rigidification (12) étant caractérisé en ce qu'il comporte un tube rigide (22) susceptible de recevoir une partie dudit extracteur flexible (11), et des moyens de connexion (13) dudit dispositif de rigidification (12) avec ledit extracteur flexible (11).

## Description

### DOMAINE TECHNIQUE

La présente invention concerne un dispositif d'extraction d'un corps étranger d'un patient, et plus particulièrement, un dispositif destiné à permettre le retrait de calculs des reins du patient.

Il concerne également un dispositif de rigidification destiné à coopérer avec un extracteur flexible, de façon à transformer cet extracteur flexible en un dispositif d'extraction rigide.

### TECHNIQUE ANTERIEURE

Comme cela est bien connu de l'homme du métier, chaque personne a habituellement un rein à droite et un autre à gauche, situés en haut et sur l'arrière de l'abdomen. Un rein mesure environ 12 cm de haut et contient des cavités (calices) pour recueillir les urines. Ces calices se réunissent pour former un bassinet. De là, un canal fin conduit les urines du rein vers la vessie : c'est l'uretère. Il mesure environ 20 cm de long. Les deux uretères (droit et gauche) conduisent les urines vers la vessie, qui est le réservoir des urines. Celles-ci s'évacuent de la vessie (miction) par un canal appelé urètre.

La présence de calculs dans un rein ou au début de l'uretère sous le rein est à l'origine d'une part de troubles ou de symptômes tels que douleurs, saignement et infection et d'autre part de risque de blocage de l'écoulement de l'urine avec à terme, l'arrêt de fonctionnement du rein, puis sa destruction. Il est donc nécessaire de retirer ces calculs. En effet, l'absence de traitement expose le patient à la persistance ou à la récidive de ces troubles et à des complications.

Il existe plusieurs méthodes de prise en charge:
- la lithotritie extracorporelle : consiste à fragmenter les calculs par des ondes de choc. Ces fragments sont alors en principe évacués par la voie naturelle. Certains calculs sont toutefois résistants aux ondes de choc ;
- Approche invasive par orifice naturels : l'urétéroscopie consiste à retirer le calcul en passant par les voies naturelles. Un dispositif muni d'un instrument de préhension est introduit dans la vessie, puis remonté dans l'uretère jusqu'au calcul. Celui-ci est pris par l'instrument et retiré du patient ;
- approche invasive transcutanée : un appareil muni d'une caméra est introduit dans le corps, directement vers le rein en traversant la peau et la paroi du dos par un seul orifice (c'est la néphrolithotomie percutanée "PNL"), ou plusieurs orifices (la laparoscopie).
- Chirurgie classique "ouverte": l'opération nécessite une ouverture large de la peau et de la paroi de l'abdomen pour aborder le rein en entier et retirer de très gros calculs.

Le choix de la technique dépend principalement de la taille, de la situation et de la dureté du calcul. En général, pour des calculs ayant une taille supérieure à 1 cm, la néphrolithotomie percutanée est préconisée.

La néphrolithotomie percutanée consiste à introduire directement dans le rein à travers la peau et la paroi du dos, un néphroscope qui permet de voir le ou les calculs, un lithotripteur qui permet de le ou les fragmenter et un extracteur à calculs qui permet de prendre ces calculs et de les extraire du patient.

Dans une intervention de néphrolithotomie conventionnelle, une sonde est mise en place dans le rein par les voies naturelles pour assurer le drainage des urines du rein. Elle sort par la vessie et l'urètre vers l'extérieur.

Le patient est ensuite placé à plat ventre, sur le dos ou sur le côté, selon les possibilités opératoires et les habitudes du chirurgien. Les cavités du rein sont ponctionnées à travers la peau du dos sous contrôle radiographique ou échographique à l'aide d'une aiguille de ponction. Puis on crée un " tunnel" d'environ 1 centimètre de diamètre entre la peau et le rein à l'aide d'une gaine d'Amplatz pour pouvoir introduire le matériel d'endoscopie (néphroscope rigide ou souple). Le néphroscope permet de visualiser directement la zone traitée à l'aide d'images transférées sur un écran vidéo. Le calcul est fragmenté en utilisant un lithotripteur ou une source laser. Le calcul est ensuite extrait en plusieurs morceaux à l'aide d'un extracteur (rigide ou souple selon le type de néphroscope employé). Dans certains cas plusieurs trajets peuvent être nécessaires. Plus précisément, la procédure commence à l'aide d'un néphroscope rigide au travers duquel un extracteur rigide est inséré. Ceci permettant un accès au calcul situé dans le prolongement du néphroscope.

Dans la pratique, il arrive fréquemment que d'autres calculs ou d'autres fragments ne soient pas positionnés dans le prolongement du néphroscope. Dans ce cas, l'urologue peut être amené à utiliser un néphroscope flexible pour explorer l'ensemble des calices et retirer les calculs résiduels. A cet effet, il va donc utiliser un extracteur flexible compatible et adapté avec le néphroscope flexible. Ces extracteurs sont fabriqués à partir de matériaux dits "à mémoire de forme" pour résister à des torsions extrêmes sans déformations.

L'intervention se termine en général par la mise en place d'une sonde dans le rein, sortant à travers la peau du dos du patient.

Dans un certain nombre de cas, il est nécessaire d'utiliser dans un premier temps, un néphroscope rigide puis, dans un deuxième temps, un néphroscope flexible, afin d'accéder à des calculs ou des fragments de calculs qui ne sont pas disposés dans le prolongement du tunnel d'entrée. Le néphroscope flexible a également l'avantage de permettre d'explorer les différents calices du rein pour détecter d'éventuels fragments.

Ceci implique d'utiliser deux instruments différents, ce qui encombre le champ opératoire, entraîne un coût en produits consommables importants et engendre une manutention importante.

Il serait donc avantageux de disposer d'un seul instrument qui bénéficie des avantages des néphroscopes rigides et des néphroscopes flexibles de telle sorte que le chirurgien puisse choisir à tout moment, l'instrument le mieux adapté à la procédure en cours.

### EXPOSE DE L'INVENTION

La présente invention se propose de résoudre les problèmes des néphroscopes de l'art antérieur en réalisant un instrument qui puisse être utilisé aussi bien dans les situations où un néphroscope rigide doit être utilisé que dans les situations où un néphroscope flexible est plus adapté.

Selon une forme de réalisation de l'invention, cet instrument peut être réversible, c'est-à-dire qu'il peut être réutilisé dans sa configuration rigide après avoir été utilisé dans sa configuration flexible et inversement.

Le but de cette invention est atteint par un dispositif d'extraction d'un corps étranger d'un patient, ce dispositif comportant :
- un extracteur flexible formé d'une gaine flexible, d'un mandrin de transmission disposé dans ladite gaine flexible, d'une poignée à laquelle est fixée une zone proximale dudit mandrin de transmission et d'un outil lié à une extrémité distale dudit mandrin de transmission;
- un dispositif de rigidification comportant un tube rigide dans lequel peut être placée la gaine flexible de l'extracteur flexible;
- des moyens de connexion de l'extracteur flexible audit dispositif de rigidification.

Le but de l'invention est également atteint par un dispositif de rigidification destiné à coopérer avec un extracteur flexible, ce dispositif de rigidification comportant un tube rigide susceptible de recevoir une partie dudit extracteur flexible, et des moyens de connexion dudit dispositif de rigidification avec ledit extracteur flexible.

Selon cette invention, un chirurgien peut utiliser le dispositif de façon conventionnelle dans deux configurations différentes. Dans une première configuration, le dispositif peut être utilisé dans un néphroscope rigide. En pratique, le chirurgien commence généralement par utiliser ce type de néphroscopes pour retirer les calculs les plus importants. Ces néphroscopes rigides ont généralement une longueur relativement courte tout comme le dispositif de l'invention, dans sa configuration rigide. De ce fait, le chirurgien peut travailler avec ce dispositif comme il a l'habitude avec les néphroscopes rigides conventionnels.

Dans une deuxième phase, si le chirurgien souhaite utiliser un dispositif flexible, en particulier dans le but d'explorer d'autres calices du rein et/ou d'en retirer des calculs ou des fragments de calculs, le dispositif de l'invention peut être transformé en extracteur flexible. Il est à noter que, de façon conventionnelle, un néphroscope flexible a une longueur plus grande qu'un néphroscope rigide. Dans une forme de réalisation préférée de la présente invention, cette caractéristique est respectée de sorte qu'un chirurgien pourra également travailler de façon conventionnelle lorsqu'il utilisera le dispositif de l'invention dans une configuration correspondant à un néphroscope flexible.

Grâce à cette invention, le chirurgien pourra utiliser un seul dispositif qui peut être flexible ou rigide, selon les besoins du moment. De plus, il pourra utiliser ce dispositif avec les appareils conventionnels, sans devoir les adapter. Il pourra également utiliser ce dispositif de la même manière que les dispositifs dont il a l'habitude, ce qui évite l'apprentissage de nouvelles manipulations et réduit les risques induits.

### DESCRIPTION SOMMAIRE DES DESSINS

La présente invention et ses avantages seront mieux compris en référence aux figures annexées et à la description détaillée d'un mode de réalisation particulier, dans lesquelles :

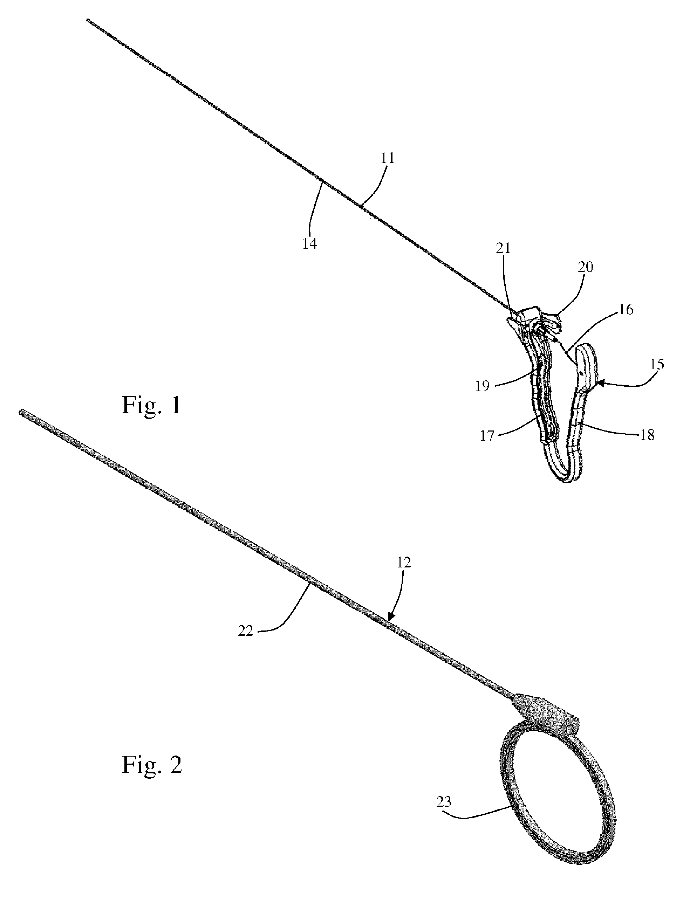

- la figure 1 est une vue en perspective d'un extracteur flexible selon la présente invention;
- la figure 2 illustre un dispositif de rigidification selon la présente invention;
- la figure 3 est une vue en perspective du dispositif d'extraction de l'invention;
- la figure 4 représente un détail d'un élément de l'invention; et
- la figure 5 représente un autre détail du dispositif de l'invention.

### MANIERE DE REALISER L'INVENTION

En référence aux figures, l'invention concerne un dispositif d'extraction 10 de corps étrangers d'un patient, et en particulier un dispositif d'extraction de calculs du corps d'un patient. Ce dispositif d'extraction est formé essentiellement de deux éléments, à savoir d'un extracteur flexible 11 et d'un dispositif de rigidification 12. Le dispositif d'extraction 10 selon l'invention comporte en outre des moyens de connexion 13 de l'extracteur flexible 11 audit dispositif de rigidification 12.

L'extracteur flexible 11 comporte une gaine flexible 14 solidaire d'une poignée 15 et un mandrin de transmission 16, également solidaire de la poignée 15. Le mandrin de transmission 16 est flexible et coulisse dans la gaine flexible 14.

La poignée 15 a une forme sensiblement similaire à un U et comporte une partie distale 17 à laquelle est fixée la gaine flexible 14 et une partie proximale 18 à laquelle est fixé le mandrin de transmission 16. Les parties distale 17 et proximale 18 de la poignée peuvent se déplacer l'une par rapport à l'autre. Selon un mode de réalisation avantageux, le déplacement relatif des deux parties de la poignée se fait grâce à l'élasticité de la matière dans laquelle cette poignée est réalisée. Cette matière peut avantageusement être un polymère tel que du polyamide, du POM (polyoxyméthylène), un polycarbonate ou de l'ABS (acrylonitrile butadiène styrène). D'autres matières adéquates pourraient également être utilisées. Il est clair que d'autres modes d'assemblages peuvent être réalisés, comme par exemple un assemblage avec une charnière dans la partie basse du U reliant la partie distale 17 et proximale 18 de la poignée. Ce mode d'assemblage permet également un déplacement relatif des parties distale et proximale de la poignée. De nombreuses variantes de la poignée peuvent être utilisées, comme par exemple une poignée avec un poussoir de type seringue.

Le mandrin de transmission 16 est solidaire, à son extrémité distale opposée à la poignée 15, d'un outil (non représenté). Selon un mode de réalisation avantageux, l'outil peut être un extracteur à calculs, permettant la préhension et l'extraction de calculs du corps d'un patient. Il est clair que d'autres outils pourraient être utilisés en fonction de l'application.

Le mandrin de transmission 16 peut prendre deux positions extrêmes, à savoir une position rentée dans laquelle l'extrémité distale du mandrin de transmission 16 de même que l'outil se trouvent à l'intérieur de la gaine flexible 14 et une position de travail dans laquelle l'outil est à l'extérieur de cette gaine 14.

Selon un mode de réalisation avantageux, la poignée 15 est conçue de telle façon que le mandrin de transmission 16 soit dans une position rentrée lorsqu'aucune force n'est exercée sur la poignée.

La poignée 15 comporte en outre une partie des moyens de connexion 13 de l'extracteur flexible 11 audit dispositif de rigidification 12. A cet effet, la partie distale 17 de la poignée comporte une fente longitudinale 19 dont l'utilité est expliquée plus en détail plus bas. La poignée 15 comporte également deux pattes élastiques 20, chaque patte élastique étant pourvue d'un ergot 21.

Selon un mode de réalisation avantageux, l'extrémité proximale du mandrin de transmission 16 est fixée de façon inamovible à la poignée 15. Il est possible d'utiliser une fixation inamovible grâce à la souplesse du mandrin de transmission 16. Ce mandrin doit toutefois être choisi de façon à ne pas subir de flambage lorsque la poignée est déplacée de la position rentrée à la position de travail tout en se pliant au niveau de la zone de fixation avec la poignée.

Dans une forme de réalisation avantageuse, la longueur de la gaine flexible 14 est telle que l'extracteur flexible 11 puisse être utilisé avec les appareils conventionnels, en particulier avec un néphroscope ou un endoscope flexible. Cette longueur est généralement comprise entre 40 et 120 cm.

Le dispositif de rigidification 12 comporte essentiellement un tube rigide 22, un organe de détournement 23 et une partie des moyens de connexion 13 de l'extracteur flexible 11 audit dispositif de rigidification 12. Le tube rigide 22 comporte un canal intérieur 24 ayant un diamètre tel que la gaine flexible 14 de l'extracteur flexible 11 puisse y être introduite. Ce tube rigide 22 a avantageusement une dimension correspondant aux néphroscopes rigides utilisés de façon conventionnelle. La longueur des néphroscopes rigides est généralement comprise entre 20 et 40 cm pour un diamètre intérieur compris entre 1 et 6 mm. Le tube rigide peut être réalisé en métal ou en polymère, le métal pouvant par exemple être un acier inoxydable de grade médical tel qu'un acier de type 316 ou un alliage approprié; le polymère pouvant par exemple être un polymère renforcé avec des fibres de carbone, de verre ou de kevlar. D'autres matériaux appropriés peuvent également être utilisés.

Dans le mode de réalisation illustré, l'organe de détournement 23 est formé d'un anneau, le canal intérieur 24 ayant une dimension suffisante pour permettre le passage de la gaine 14 de l'extracteur flexible 11.

La longueur de l'anneau, ou le périmètre de l'organe de détournement 23 lorsque celui-ci a une forme circulaire, est sensiblement égale à la différence entre la longueur du tube rigide 22 et la longueur de la gaine flexible 14, comme cela est expliqué plus en détail plus bas.

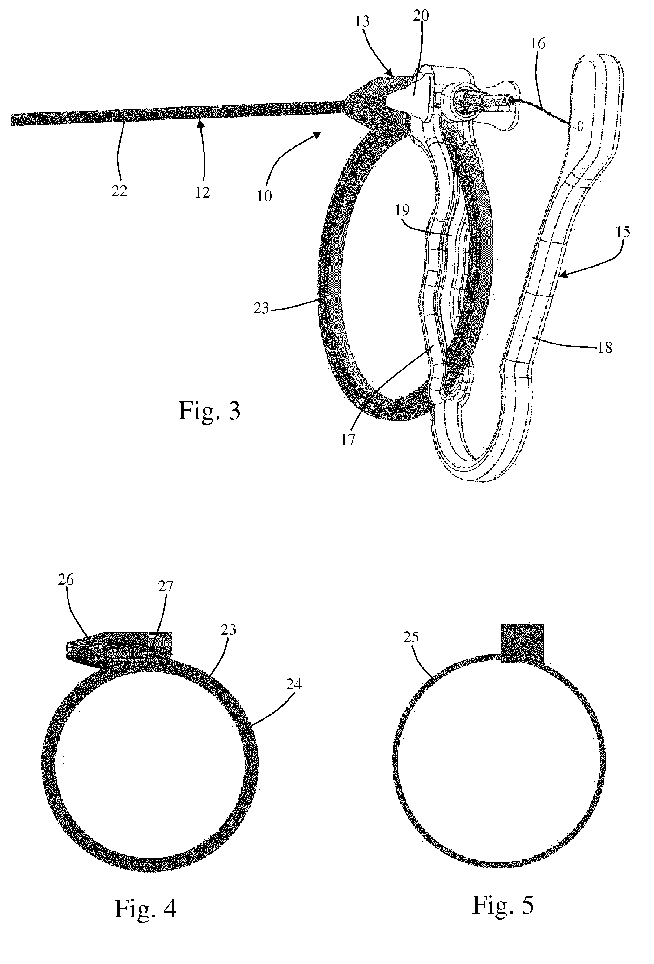

L'organe de détournement 23 tel qu'illustré dans les figures 3 à 5 comporte un couvercle 25 permettant d'accéder au canal intérieur 24. Ceci peut faciliter la mise en place de la gaine flexible 14 dans cet organe de détournement 23.

Selon un mode de réalisation avantageux, l'organe de détournement 23 a une épaisseur et une position telles qu'une partie de cet organe puisse être placé dans la fente longitudinale 19 de la poignée 15. Il est à noter que cet organe de détournement pourrait être placé dans une position séparée de la poignée 15, de sorte que ces deux éléments ne soient pas en contact l'un avec l'autre.

Le dispositif de rigidification 12 comporte un organe de maintien 26 dans lequel le tube rigide 22 est fixé. Cet organe de maintien 26 est muni d'encoches 27 agencées pour coopérer avec les ergots 21 de la poignée 15.

Comme cela est bien connu de l'homme du métier, lorsqu'un chirurgien cherche à retirer un corps étranger d'un patient, et en particulier un calcul d'un rein du patient, il peut utiliser une technique de néphrolithotomie percutanée comme cela est indiqué plus haut. Selon cette technique, un canal est introduit par le dos du patient jusqu'au rein duquel le calcul doit être retiré. Dans la majorité des cas, le chirurgien utilise un néphroscope rigide pour accéder aux calculs situés approximativement dans le prolongement du néphroscope. Pour les calculs disposés ailleurs que dans le prolongement du néphroscope, par exemple dans d'autres calices du rein, le chirurgien utilise un néphroscope souple.

Selon la présente invention, le dispositif d'extraction 10 peut tout d'abord être utilisé comme un néphroscope rigide conventionnel. A cet effet, la gaine flexible 14 est tout d'abord introduite dans le canal intérieur 24 de l'organe de détournement 23. Pour réaliser ceci, la gaine flexible peut simplement être poussée dans l'organe de détournement 23, sa forme étant telle que la gaine ne rentre pas directement dans le tube rigide 22, mais passe tout d'abord dans le canal intérieur 24 avant de ressortir de ce canal et de rentrer dans le tube rigide 22. Si l'organe de détournement 23 comporte un couvercle 25, celui-ci peut éventuellement être retiré, si nécessaire, pour faciliter la mise en place de la gaine flexible 14.

Cette gaine flexible 14 est ensuite poussée jusqu'à sa position extrême dans laquelle l'outil placé à l'extrémité du mandrin de transmission 16 de l'extracteur flexible 14 est disposé à proximité de l'extrémité libre du tube rigide 22.

Dans cette position, les différentes pièces formant les moyens de connexion 13 sont engagées de façon à maintenir l'extracteur flexible solidaire du dispositif de rigidification. De façon plus détaillée, l'organe de détournement 23 du dispositif de rigidification 12 est engagé dans la fente longitudinale 19 de la poignée 15 de l'extracteur flexible. Les ergots 21 formés sur les pattes élastiques 20 de la poignée 15 sont engagés dans les encoches 27 de l'organe de maintien 26. De cette façon, l'extracteur flexible 11 et le dispositif de rigidification 12 sont solidaires et peuvent être utilisés de la même manière qu'un extracteur rigide conventionnel. L'actionnement de la poignée 15 a pour effet de déplacer le mandrin de transmission 16, ce qui permet un actionnement de l'outil disposé à l'extrémité de ce mandrin de transmission. L'élasticité de la poignée 15 est telle qu'en l'absence de force appliquée à cette poignée, l'outil est disposé à l'intérieur de la gaine flexible 14 et à l'intérieur du tube rigide 22.

Lorsque le chirurgien souhaite utiliser le dispositif d'extraction 10 dans un néphroscope souple, il écarte les pattes élastiques 20, ce qui a pour effet de libérer les ergots 21 de l'organe de maintien 26. Il extrait l'organe de détournement 23 de la fente longitudinale 19 de la poignée 15 et il retire la gaine flexible 14 du tube rigide 22. Dans cette configuration, la totalité de la longueur de la gaine flexible 14 peut alors être utilisée, ce qui permet d'utiliser cet extracteur 11 avec des néphroscopes souples conventionnels.

Dans le cas où le chirurgien souhaiterait réutiliser le dispositif d'extraction 10 dans sa configuration d'extracteur rigide, il suffit de réintroduire la gaine flexible 14 dans l'organe de détournement 23, de replacer cet organe de détournement 23 dans la fente longitudinale 19 de la poignée 15 et les ergots 21 sur l'organe de maintien 26.

La présente invention permet donc de disposer, avec un seul dispositif d'extraction, d'une configuration correspondant à un néphroscope souple et une configuration correspondant à un néphroscope rigide. Dans le mode de réalisation décrit, ce dispositif est totalement réversible, ce qui permet au chirurgien de choisir à n'importe quel moment, quelle est la configuration la plus adaptée à la situation. Le dispositif selon l'invention est également dimensionné de façon à correspondre aux dimensions des néphroscopes flexibles ou rigides conventionnels, ce qui permet d'utiliser le dispositif de l'invention avec des appareils conventionnels, sans que d'une part, d'autres appareils doivent être développés et sans que d'autre part, le chirurgien doivent s'habituer à d'autres instruments ou d'autres manières de travailler. Il est toutefois possible de prévoir un dispositif d'extraction ne comportant pas d'organe de détournement 23. Dans ce cas, la longueur du dispositif de l'invention est sensiblement la même, dans sa configuration flexible et dans sa configuration rigide.

Dans l'exemple illustré, les moyens de connexion 13 sont formés notamment de la poignée 15 et du dispositif de détournement 23. Il est également possible que cette poignée et ce dispositif de détournement soient indépendants l'un de l'autre et ne fassent pas partie des moyens de connexion.

Il est également possible d'utiliser des moyens de connexion non réversibles. Dans ce cas, les moyens de connexion peuvent par exemple être brisés selon une zone de rupture, de façon à séparer le dispositif de rigidification de l'extracteur flexible, sans possibilité de reconnecter le dispositif de rigidification à cet extracteur flexible.

La présente invention a été décrite dans une application dans laquelle des calculs sont retirés des reins d'un patient. Le même principe pourrait être appliqué à d'autres corps étrangers à retirer d'un patient.

## Revendications

1. Dispositif d'extraction (10) de corps étrangers d'un patient, ce dispositif étant **caractérisé en ce qu'**il comporte :
• un extracteur flexible (11) formé d'une gaine flexible (14), d'un mandrin de transmission (16) disposé dans ladite gaine flexible (14), d'une poignée (15) à laquelle est fixée une zone proximale dudit mandrin de transmission (16) et d'un outil lié à une extrémité distale dudit mandrin de transmission (16);
• un dispositif de rigidification (12) comportant un tube rigide (22) dans lequel peut être placée la gaine flexible (14) de l'extracteur flexible (11); et
• des moyens de connexion (13) de l'extracteur flexible (11) audit dispositif de rigidification (12).

2. Dispositif d'extraction selon la revendication 1, **caractérisé en ce que** le mandrin de transmission (16) est coulissant dans ladite gaine flexible (14) et **en ce que** la zone proximale dudit mandrin de transmission (16) est solidaire de ladite poignée (15).

3. Dispositif d'extraction selon la revendication 1, **caractérisé en ce que** la poignée (15) comporte une partie distale (17) et une partie proximale (18), ces parties distale et proximale étant mobiles l'une par rapport à l'autre.

4. Dispositif d'extraction selon la revendication 1, **caractérisé en ce que** la poignée (15) comporte une fente longitudinale (19), cette fente longitudinale faisant partie desdits moyens de connexion (13).

5. Dispositif d'extraction selon la revendication 1, **caractérisé en ce que** les moyens de connexion (13) comportent au moins une patte élastique (20) solidaire de la poignée (15).

6. Dispositif d'extraction selon la revendication 5, **caractérisé en ce que** ladite patte élastique (20) est munie d'au moins un ergot (21) coopérant avec ledit dispositif de rigidification (12).

7. Dispositif d'extraction selon la revendication 1, **caractérisé en ce que** le dispositif de rigidification (12) comporte un tube rigide (22) susceptible de recevoir la gaine flexible (14) de l'extracteur flexible (11).

8. Dispositif d'extraction selon la revendication 1, **caractérisé en ce que** le dispositif de rigidification (12) comporte un organe de détournement (23) agencé pour recevoir une partie de la gaine flexible (14) de l'extracteur flexible (11).

9. Dispositif d'extraction selon la revendication 1, **caractérisé en ce que** le dispositif de rigidification (12) comporte un organe de maintien (26) solidaire du tube rigide (22) et comportant une partie desdits moyens de connexion (13) de l'extracteur flexible (11) audit dispositif de rigidification (12).

10. Dispositif d'extraction selon la revendication 9, **caractérisé en ce que** l'organe de maintien (26) comporte au moins une encoche (27) agencée pour coopérer avec ledit ergot (21) de la patte élastique (20).

11. Dispositif d'extraction selon les revendications 4 et 8, **caractérisé en ce que** l'organe de détournement (23) est positionné et dimensionné de manière à se placer dans la fente longitudinale (19) de la poignée (15) lorsque l'extracteur flexible (11) est monté dans le dispositif de rigidification (12).

12. Dispositif d'extraction selon la revendication 8, **caractérisé en ce que** l'organe de détournement (23) comporte un canal intérieur (24) susceptible de recevoir une partie de la gaine flexible (14) de l'extracteur flexible (11).

13. Dispositif de rigidification (12) destiné à coopérer avec un extracteur flexible (11), ce dispositif de rigidification (12) étant **caractérisé en ce qu'**il comporte un tube rigide (22) susceptible de recevoir une partie dudit extracteur flexible (11), et des moyens de connexion (13) dudit dispositif de rigidification (12) avec ledit extracteur flexible (11).

14. Dispositif de rigidification selon la revendication 13, **caractérisé en ce qu'**il comporte en outre un organe de détournement (23) agencé pour recevoir une partie dudit extracteur flexible (11).

15. Dispositif de rigidification selon la revendication 14, **caractérisé en ce que** ledit organe de détournement (23) est formé d'un anneau comportant un canal intérieur (24).

16. Dispositif de rigidification selon la revendication 14, **caractérisé en ce que** ledit organe de détournement (23) coopère avec une poignée (15) de l'extracteur flexible (11) de manière à rendre solidaires, ledit dispositif de rigidification (12) et l'extracteur flexible (11).

17. Dispositif de rigidification selon la revendication 14, **caractérisé en ce que** l'extracteur flexible (11) et l'organe de détournement (23) ont une longueur relative telle que lorsque l'extracteur flexible (11) est introduit dans le dispositif de rigidification (12), l'ensemble a une longueur compatible avec une utilisation comme néphroscope rigide.
